# EUROPEAN PATENT SPECIFICATION

(11) **EP 1 565 196 B1**
(45) Date of publication and mention of the grant of the patent: **19.09.2007**
(21) Application number: 03774329.1
(22) Date of filing: 26.11.2003
(51) Int. Cl.: A61K 35/00, A61P 35/00

(54) **PREPARING METHOD OF DENDRITIC CELL FOR CANCER IMMUNOTHERAPY**
VERFAHREN ZUR HERSTELLUNG EINER DENDRITISCHEN ZELLE FÜR DIE KREBSIMMUNOTHERAPIE
PROCÉDÉ DE PRÉPARATION D'UNE CELLULE DENDRITIQUE POUR L'IMMUNOTHÉRAPIE ANTICANCEREUSE

(30) Priority: 28.11.2002 KR 20020074834
(43) Date of publication of application: 24.08.2005
(73) Proprietor: Binex Co., Ltd., Busan 604-846 (KR); Lee, Baek-Chung, 609-841 Busan (KR)
(72) Inventor: KANG, Chi-Dug, Seodaesindong 3-ga 602-753 Busan (KR); JEONG, Min-Ho, Suycong-gu Busan, 613-763 (KR)
(74) Representative: Kuhnen & Wacker
(86) International application number: PCT/KR2003/002572
(87) International publication number: WO 2004/047849

(56) References cited:
- WO-A1-93/20186
- WO-A1-97/12633
- WO-A2-01/35989
- WO-A2-01/36487
- US-A- 5 788 963
- FRIEDMAN: "Immune modulation by ionizing radiation and its implications for cancer immunotherapy" CURRENT PHARMACEUTICAL DESIGN, vol. 8, September 2002 (2002-09), pages 1765-1780, XP008058094
- NIKITINA ET AL: "Combination of gamma-irradiation and dendritic cell administration induces a potent antitumor response in tumor-bearing mice: Approach to treatment of advanced stage cancer" INTERNATIONAL JOURNAL OF CANCER, vol. 94, 2001, pages 825-833, XP002362369
- VAN MEIRVENNE ET AL: "Efficient genetic modification of murine dendritic cells by electroporation with mRNA" CANCER GENE THERAPY, vol. 9, September 2002 (2002-09), pages 787-797, XP002362106
- NISHIOKA ET AL: "Genetic modification of dendritic cells and its application for cancer immunotherapy" THE JOURNAL OF MEDICAL INVESTIGATION, vol. 49, February 2002 (2002-02), pages 7-17, XP002362370

## Description

### Technical Field

The present invention relates to a cancer immunotherapy, and in particular to a cancer immunotherapy using a dendritic cell that is known as a new type dendritic cell-based cancer immunotherapy implemented based on a method in which a dendritic cell is injected into a tumor after radioactive rays are scanned in accordance with a known dendritic cell-based cancer immunotherapy using a total cell lysates as a source of antigen, and a method in which an antigen is loaded into a dendritic cell based on an electroporation method. In particular, the invention relates to a method of preparing dendritic cells using electroporation and pulse loading.

### Background Art

Recently, it is difficult to overcome the basic problems in death due to cancer for the reasons that there are a side effect in a conventional cancer therapy, and a tolerance due to a genetic instability. In the case of recrudescence of cancer, there is not known any therapy.

Therefore, an anti-tumor vaccine development is needed, which is implemented based on an immunity checking system. So far, there are known a combined peptide vaccine, gene vaccine, thermal impact protein vaccine, etc. Among the above methods, it is known that the dendritic cell method is most effective. The dendritic cells are the strongest and important antigen presenting cells in a live body, so that the dendritic cells are well adapted to the anti-tumor vaccine and almost diseases related to the immune reaction.

However, the immunotherapy using the dendritic cell is not standardized. Some research centers are trying to maximize the effects and to implement an easier adjustment. It is well known that the immune system is capable of recognizing tumors and attacking the same. Both humoral immunity and cell-mediated immunity are related to the dissolution of all tumors, but in almost case the regression of the tumors through a particular recognition of the tumor antigen is related to a cell-mediated immunity. In particular, the operations that CD8+cytotoxic T lymphocyte (CTL) recognizes a tumor cell and kills the same are proved through various models. (1. Boon, T., J.C. Cerottini, B. Van den Eynde, P. van der Bruggen, and A. Van Pel. 1994. Tumor antigens recognized by T lymphocytes. Annu Rev Immunol 12:337. 2. Celluzzi, C.M., J.I. Mayordomo, W.J. Storkus, M.T. Lotze, and L.D. Falo, Jr. 1996. Peptide-pulsed dendritic cells induce antigen-specific CTL-mediated protective tumor immunity. J Exp Med 183, 1:283. 3. Feltkamp, M.C., H.L. Smits, M.P. Vierboom, R.P Minnaar, B.M. de Jongh, J.W. Drijfhout, J. ter Schegget, C.J. Melief, and V.M. Kast. 1993. Vaccination with cytotoxic T lymphocyte epitope-containing peptide protects against a tumor induced by human papillomavirus type 16-transformed cells. Eur J Immunol 23, 9:2242.3). The dendritic cell is a unique antigen-presenting cell that may induce an immunity reaction and is widely used for curing cancer and an antigen-particular immunotherapy of infection diseases. (Banchereau, J., F. Briere, C. Caux, J. Davoust, S. Lebecque, Y.J. Liu, B. Pulendran, and K. Palucka. 2000. Immunobiology of dendritic cells. Annu Rev Immunol 18:767.) For modification of murine dendritic cells by electroporation, see Van Meirvenne et al, Cancer Gene Therapy ; 2002, pages 787-797. The ability that the dendritic cell is initialized so that the T cell recognizes and kills the tumor cells based on the tumor-particular method is reported in various animal models. In addition, the immunization induced based on the dendritic cell may induce an immunity memory for thereby preventing the occurrence of tumors.

The dendritic cell occupies only about 0.3% in the entire circulation leukocyte and is formed of a heterogeneous group having a certain expression type different from macrophage. The dendritic cell is a strong antigen-presenting cell that is largely different from the B cell having a relatively weak antigen-representing ability or macrophage cell. The dendritic cell is induced from a monocyte pulsed by IL-4 and GM-CSF or a monocyte precursor (CD34+ cell) and is used for vaccine protocol. The immature dendritic cell extracts apoptotic or necrotic, bacteria and a certain antigen or an antigen epitope from a water soluble protein. The antigens induced into the dendritic cells through various procedures of apoptotic body capture, macropinocytosis, and mannose receptor or endocytosis, etc. through CD32 or CD64 are known to form a complex with a major histocompatibility complex through a series procedure.

The captured antigen is carried with a MHC class II section and is treated and is proposed as a peptide-MHC complex for thereby inducing a CD4+ T cell reaction. Recently, it was proved that the dendritic cell has ability for representing through the MHC I molecular by the proteasome and TAP dependence with respect to the antigen induced from the outside. Therefore, the dendritic cells are judged to pulse the T cell and B cell through a special treatment procedure with respect to an external antigen. The dendritic cell is widely used for the cancer immunity therapy based on the above-described characteristics.

The antigen that is most generally used during the dendritic cell cancer immunotherapy is epitope peptide. However, it is needed to check whether the above antigen is presented in a carcinoma of a patient. The above antigen may be used for only a patient who has a particular HLA type that may form a complex body. In addition, there is not much peptide in the US and Europe. In Korea, it is known that there is not provided the tumor antigen confirmed. Furthermore, the antigens of digestive cancer and respiratory organ those are the targets of the researches of the present invention are not introduced yet. Searching the tumor antigen needs long time. In Korea, it is impossible to use the peptide antigen for a dendritic cell cancer immunotherapy.

In order to avoid the above problems, there are several trials for using the unidentified TAA (total cell lysates, apoptotic/necrotic cells, total mRNA, tumor cells fused with DC, etc.) as an antigen. However, in this case, there are problems for loading the unidentified TAA on the dendritic cell. The total cell lysates is known to generally pulse CD4+ helper T cell. There is inconvenience for directly separating mRNA from a tumor tissue or cell. The apoptotic cell should be disadvantageously cultivated together with the dendritic cell after a cancer cell is separated and cultivated, and an apoptosis is formed. The confusion of the cancer cell and dendritic cell needs a cancer cell separation cultivation from the tumor tissue. In this research, the above disadvantages are researched in cooperation with the ministry of health and welfare and the Korean science foundation. As a result of the researches, a new method of dendritic cell cancer immunotherapy such as a method for injecting a dendritic cell into tumor to which radioactive rays are irradiated and a method for loading an antigen into a dendritic cell based on electroporation is developed.

According to a result of the survey on the 2000 year cancer registration business reported by the ministry of health and welfare and the national cancer center, among the total cancer numbers, the stomach cancer and cervical cancer that are the developing country type cancers are gradually decreased, and the large intestine cancer, breast cancer and lung cancer that are the advanced country type cancers are gradually increased. The total cancer occurrence numbers registered in 2000 is 83,846 showing an increase by 1.9% compared to 1999. Among the cancers, the number of the digestive organ cancer is top. Namely, in the case of male, the stomach cancer, liver cancer and large intestine cancer are 24.5%, 16.3% and 10.2%, respectively, that are the half of the total cancers, and in the case of female, the stomach cancer and large intestine cancer are 15.8% and 10.5%. The lung cancer in the respiratory cancer is significantly increased, and ranks the second place (11.5%) next to the stomach cancer among the total cancers. In the cancer death ratio, the lung cancer is the top.

Even though a cancer earlier detection based on a development of diagnosis technology, an increasing cancer prevention trend and operation, a radioactive curing and anti-cancer chemical therapy are developed, the cancer occurrence rate and the death ratio due to cancer are not decreased, so that it is urgently needed to induce an improvement in a conventional cancer therapy or a new therapy technique. In the case of a surgical operation or radioactive ray therapy that is classical type cancer therapy methods, a remote micro transit affection may remain. There may be a side effect due to a hematosis function limit may occur due to destruction of marrow. In the case that a tolerance with respect to a carcinostatis substance of the tumor cell is generated, a desired curing effect is not obtained. Therefore, a method for minimizing the therapy resistance and side effect and preventing a minimal residual lesion is capable of significantly decreasing the death rate due to cancer by preventing a relapse of cancer. In this point, the immunotherapy, namely the dendritic cell-based cancer immunotherapy is known to provide a possibility as the most effective anti-tumor vaccine.

### Disclosure of Invention

Accordingly it is an object of the present invention to provide a method for preparing dendritic cells having enhanced anti-tumor immunity by loading protein-based tumor lysates as tumor antigens into immature dendritic cells using electroporation and pulse loading.

### Brief Description of Drawings

The present invention will become better understood with reference to the accompanying drawings which are given only by way of illustration and thus are not limitative of the present invention, wherein;

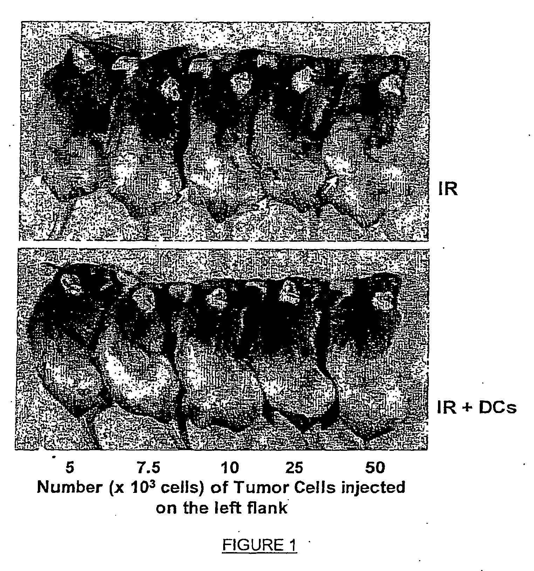

Figure 1 is a view illustrating a therapy effect. As shown therein, no therapy effect was observed in the case that only radioactive rays were irradiated on a tumor of a right side femoral region when a tumor therapy effect was observed in a group IR in which radioactive rays were irradiated to a tumor, and a group (IR + DCs) in which a homogeneous dendritic cell was injected after radioactive rays were irradiated to a tumor. It was possible to observe a significant therapy effect in the case that a dendritic cell was injected into a left side back after radioactive rays were irradiated to a tumor of a right side femoral region. The IR group is a group in which only radioactive ray irradiation is performed, and IR+DCs is a group in which a radioactive ray is irradiated, and a dendritic cell is injected to the opposite side. The IR group does not have a significant effect. The tumor of the right femoral region is significantly decreased after IR+DCs. Induction of the therapeutic anti-tumor immunity against distance tumor by intratumorial injection of dendritic cells into the irradiated tumor. Photographs (B) were taken after last immunization

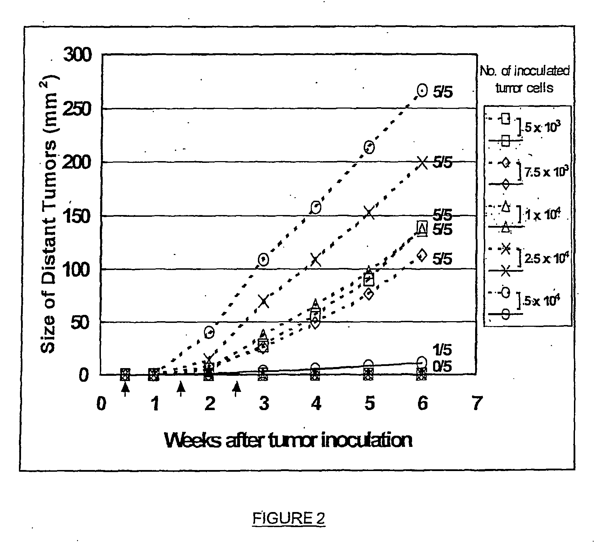

In Figure 2, a solid cancer was provided by its concentration, and the size of growing solid cancer was compared (blue color). In a test group, a solid cancer was formed and was treated by radioactive rays. An immature dendritic cell was provided. The size of the tumor whose growth was inhibited was measured (red color). As a result, a desired effect was obtained. All samples were cured after 6 weeks except for one sample in the test group to which the maximum concentration tumor cell was provided, so that solid cancer was not observed. In the case of the remaining one sample, the average size of 250mm² was significantly decreased to the size of 50mm².

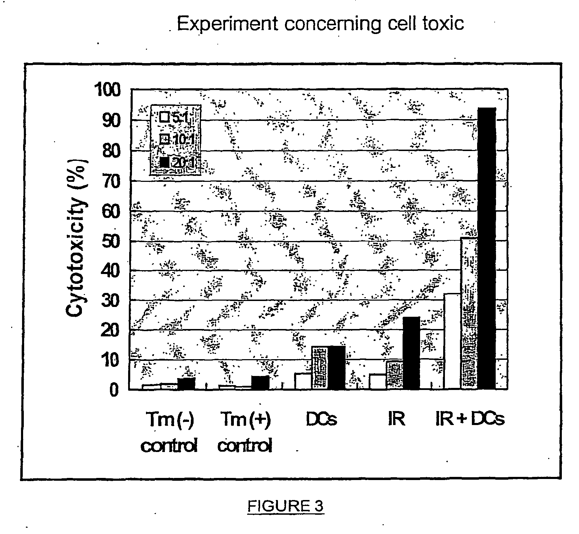

In Figure 3, the test was conducted by dividing the test group (IR + DCs) into a tumor (Tm (-)) without tumor, a tumor comparison group (Tm(+)), a dendritic cell treatment group (DCs), a radioactive ray-irradiated group (IR), and a dendritic cell treatment group irradiation with radioactive rays. The most effective cytotoxicity was shown in the IR + DCs group, so that it was known that a cancer immunotherapy was high. 5:1, 10:1 and 20:1 represent ratios of the Splenocytes: Tumor cells. Enhancement of anti-tumor immunity by intralesional injection of dendritic cells (DC) into irradiated (IR) tumor

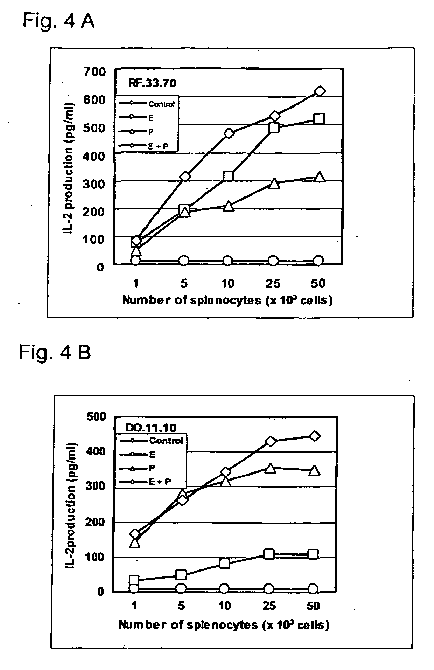

Figures 4A and 4B show the MHC class I (A) and II (B) presentation of OVA by dendritic cells after electroporation (E), pulse (P), and combination of both (E+P).

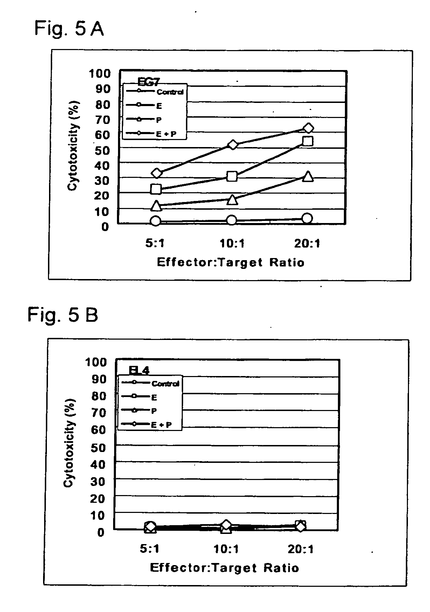

Figures 5A and 5B exemplify the induction of OVA-specific cytotoxic activity by dendritic cells, into which OVA was loaded by electroporation (E), pulse (P), and combination of both (E+P).

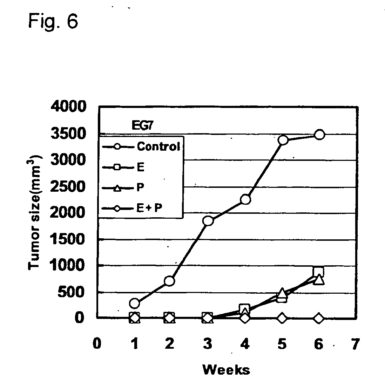

Figure 6 exemplifies the OVA-specific preventive anti-tumor immunity induced by immunization with DCs, into which OVA was loaded by electroporation (E), pulse (P) or both (E+P).

As a result of the experiments performed by the present researchers, in the cell toxic experiment using a dendritic cell, a mature dendritic cell stimulates immunity in such a manner that an immature dendritic cell is accurately injected into a tumor to which radioactive rays are irradiated. As a result of the performance experiment with respect to the tumor cells in the immunity by the immunity stimulation, the cytotoxicity is significantly increased. In addition, in the case that the mature dendritic cell in which a tumor antigen is loaded externally, it is possible to observe a significant increase of interleukin-2. In addition, the above effect is obtained as a result of the experiment performed with respect to the solid tumor.

### [Examples]

The actual experiments according to the present invention will be described in detail.

In the following experiments, the experiment was conducted using a dendritic cell as a cancer immunotherapy. As a result of the experiment, the anti-tumor effects and interleukin-2 with respect to the solid cancer are increased.

### [Example 1]

The MCA102 fibrosarcoma cell was injected under the skin of the right femoral region of a syngeneic C57BU6 mouse (6~8 weeks, female), and the tumor was grown to a diameter of 8mm. The femoral region having the tumor is placed in the radioactive ray irradiation region of the Linac radioactive ray irradiation apparatus (Siemens mevatron 67), and then the 15Gy radioactive rays were irradiated into the homogeneous dendritic cells in the tumor. The above processes were performed three times per one time per week. After one week, the splenocyte was separated from the mouse of a group a comparison group without tumor, a group with a tumor, a group that the homogenous dendritic cell was injected into the tumor to which the radioactive rays were not irradiated, a group that the radioactive rays were irradiated to the tumor, and a group that the radioactive rays were irradiated, and the homogeneous dendritic cells were irradiated, and then the cytotoxicity was measured with respect to the tumor cells. A certain amount of the tumor cells was processed at the magnitude of 5, 10 and 20 times with respect to the splenocytes, and the relative cell toxic was observed.

As a result, in the group having only the tumor, the cytotoxicity was not shown like the comparison group without the tumor. A small amount of the cytotoxicity was shown in the group that the homogenous dendritic cell was injected into the tumor to which the radioactive rays were not irradiated, and the group that the radioactive rays were irradiated to the tumor. However, in the group that the radioactive rays were irradiated and the homogeneous dendritic cells were injected, the cytotoxicity was significant differently from the other groups. Therefore, it was known that it is possible to significantly increase the anti-tumor immunity by injecting the dendritic cells into the tumor to which the radioactive rays were irradiated (refer to Figure 3).

In addition, the tumor therapy effect was checked in the opposite sides in the group that the radioactive rays were irradiated to the tumor, and the group that the radioactive rays were irradiated, and the homogeneous dendritic cells were injected. In the case that the radioactive rays were irradiated into the tumor of the femoral region of the right side, the therapy effects were not observed. In the case that the radioactive rays were irradiated to the tumor of the right side femoral region, and then the dendritic cells were injected, a significant therapy effect was visually observed (refer to Figure 1). The solid cancers were processed by the concentration, and the sizes of the solid cancers were compared (blue color). In the experiment group, the solid cancers were formed and irradiated by the radioactive rays, and then the immature dendritic cells were injected, and the sizes of the tumors whose growth was inhibited were compared (red color). As a result, the effects were significant. After six weeks, all mice were cured except for one mouse to which the tumor cells were injected in maximum, and the solid cancer was not found. In the case of one mouse still having the tumor, the size of the average 250mm² were decreased to below 50mm² (refer to Figure 2).

### [Example 2]

There are prepared the group E in which OVA (4mg/ml) was electroporated, and the OVA was washed and cultivated for 16 hour, the group P in which the OVA (4mg/ml) was pulsed for 16 hours, and the group E+P that the OVA was electroporated and was pulsed for 16 hours. The IL-2 was measured in the RF33.70 (MHC class I) or D0.11.10 (MHC class II) cells using the dendritic cells of the above three groups. As a result of the measurement, the group E effectively provided the OVA to the MHC class I. The group E+P provided the OVA to the MHC class I at a larger amount than that of the group E (Figure 4A). As expected, the group P provided more OVA to the MHC class II compared to the group E, and the group E+P provided more OVA to the MHC class II compared to the group P (Figure 4B). As a result, it was possible to more effectively provide the antigen to the MHC class I by loading the antigen into the dendritic cells by electroporation. It was possible to provide more antigen to the MHC class II. Therefore, it was expected that the electroporation was capable of effectively increasing the immunity. (Refer to Figs. 4A and 4B)

### [Example 3]

In a result of the cytotoxicity of the splenocyte with respect to the EG7 cell (EL4 cells transfected with an OVA cDNA) when the OVA was pulsed to the homogeneous dendritic cells based on the electroporation, if the electroporation more effectively presented the OVA to the MHC class I, when the C57BL/6 mouse was decreased to the OVA-electroporated, it was checked whether a specific immunity was generated compared to when it is decreased based on the OVA-pulsed DC. There were prepared the group E that the OVA was electroporated, and the OVA was washed and cultivated for 16 hours, the group that the OVA was pulsed for 16 hours, and the group E+P that the OVA was electroporated, and the OVA was pulsed for 16 hours. The dendritic cell (1×10⁶ cells) was injected under the skin of the right waist of the C57BU6 mouse totally three times for one time per week. After one week, the slenocytes were separated, and the cytotoxicity was observed with respect to the EG7 cell (EL4 cells transfected with an OVA cDNA). As a result, the group E had the stronger cell toxicity compared to the group P. The cell toxicity of the group E+P was stronger than the group E (Figure 5A). When the above experiment was performed with respect to the EL4 cell, the cell toxicity was not observed in all groups (Figure 5B). As a result, the cell toxicity with respect to the EG7 was unique with respect to the OVA. It was known that the electroporation increased the antigen presentation to the MHC class I for thereby effectively increasing the immunity. (Refer to Figs. 5A and 5B).

The mouse was processed in the above manner. After one week, the EG7 and EL4 cells were injected into the left back, and then the tumor occurrence inhibition effect was checked. As a result, it was possible to obtain a certain tumor inhibition effect similar in both the groups E and P. In the group E+P, the tumor occurrence inhibition effects were very high (Figure 6). In addition, the above experiment was performed with respect to the EL4 cell, the tumor occurrence inhibition effects were not observed in all groups. (Refer to Fig. 6)

### Industrial Applicability

As described above, cancer immunotherapy using the dendritic cells of the claimed method is effective for curing cancers. The present invention may be well adapted to all kinds of solid cancer therapy using the dendritic cell-based cancer immunotherapy newly developed in the present invention. It is possible to accurately load an immature dendritic cell into an intra-lesion in the tumor to which radioactive rays irradiated, with respect to the patients who do not have cancer tissue.

## Claims

1. A method for preparing dendritic cells having enhanced anti-tumor immunity by loading protein-based tumor lysates as tumor antigens into immature dendritic cells, the method comprising the steps of:
a) loading tumor lysates into immature dendritic cells by electroporation in order to increase antigen presentation by MHC class I, such that the dendritic cells can stimulate cytotoxic T lymphocytes; and
b) additionally loading tumor lysates into the dendritic cells by pulse in order to increase antigen presentation by MHC class II, such that the dendritic cells can stimulate helper T cells.

## Patentansprüche

1. Verfahren zum Herstellen dendritischer Zellen mit gesteigerter anti-Tumor-Immunität, durch Einbringen Protein-basierter Tumorlysate als Tumorantigene in unreife dendritische Zellen, wobei das Verfahren die Schritte umfasst:
a) Einbringen von Tumorlysaten in unreife dendritische Zellen durch Elektroporation, um die Antigen-Präsentation durch die MHC-Klasse I zu steigern, so dass die dendritische Zelle cytotoxische T-Lymphocyten stimulieren kann; und
b) zusätzliches Einbringen von Tumorlysaten in dendritische Zellen durch einen Puls, um die Antigen-Präsentation durch die MHC-Klasse II zu steigern, so dass die dendritische Zelle T-Helferzellen stimulieren kann.

## Revendications

1. Procéde de préparation des cellules dendritiques ayant une immunité antitumorale améliorée en chargeant des lysats tumoraux à base de protéine comme antigènes tumoraux dans des cellules dendretiques immatures, le procédé comprenant les étapes suivantes :
a) charger des lysats tumoraux dans des cellules dendritiques immatures par électroporation afin d'augmenter la présentation d'antigène par le CMH de classe I, de telle manière que les cellules dendritiques puissent stimuler les lymphocytes T cytotoxiques ; et
b) charger en outre des lysats tumoraux dans les cellules dendritiques par impulsion afin d'augmenter la présentation d'antigène par CMH de classe II, de telle manière que les cellules dendritiques puissent stimuler les cellules T auxiliaires.
